Europäisches Patentamt

European Patent Office

Office européen des brevets

(19)

(11) Veröffentlichungsnummer: **0 167 661**

**A1**

(12) **EUROPÄISCHE PATENTANMELDUNG**

(21) Anmeldenummer: **84111399.6**

(22) Anmeldetag: **25.09.84**

(51) Int. Cl.⁴: **B 65 D 47/08**
**E 05 D 1/02**

(30) Priorität: **28.06.84 DE 3423851**

(43) Veröffentlichungstag der Anmeldung:
**15.01.86 Patentblatt 86/3**

(84) Benannte Vertragsstaaten:
**AT BE CH DE FR GB IT LI NL SE**

(71) Anmelder: ZELLER PLASTIK Koehn, Gräbner & Co.
Auf dem Barl Postfach 1120
D-5583 Zell/Mosel(DE)

(72) Erfinder: Brach, Ulrich
Königsbergweg 10a
D-5583 Zell/Mosel(DE)

(74) Vertreter: Schroeter, Helmut et al,
Bocksgasse 49
D-7070 Schwäbisch Gmünd(DE)

(54) Schnappscharnier aus Kunststoff.

(57) Ein einteiliges Schnappscharnier aus Kunststoff mit zwei
Scharnierteilen (4,2) die durch ein Hauptgelenk miteinander
verbunden sind. Ein federndes Verbindungsstück (10) ist an
seinen Enden durch Nebengelenke mit den Scharnierteilen
verbunden, wobei die Nebengelenke (14,12) quer zu den
Gelenkachsen einen Abstand vom Hauptgelenk (6) haben
und die Gelenke als Filmscharniere ausgebildet sind. Die
Scharnierteile haben die Tendenz, aus einer Stellung labilen
Gleichgewichts innerhalb ihres Schwenkbereiches in die
eine oder andere Endstellung zu schnappen. Das Schnappscharnier ist ein gesonderter Bauteil. Der eine Scharnierteil
(4) trägt ein Verschließelement (32) für eine Entnahmeöffnung (30) der andere Scharnierteil (2) mindestens einen
Steckteil (16) mit Rastelementen (18,56). Hiermit kann das
Schnappscharnier an einem Behälter oder an dessen Verschlußstück befestigt werden.

FIG. 3

## SCHNAPPSCHARNIER AUS KUNSTSTOFF

### Stand der Technik, Aufgabe, Lösung

Die Erfindung bezieht sich auf ein einteiliges Schnappscharnier aus Kunststoff mit den im Oberbegriff von Anspruch 1 genannten Merkmalen. Derartige Schnappscharniere sind bekannt aus der DE-PS 18 13 187 und der DE-OS 18 08 875. Diese Schnappscharniere gestatten es dem frei beweglichen Scharnierteil, entweder in eine Schließstellung oder in eine Offenstellung zu schnappen und in dieser Stellung dann stehenzubleiben.

In den beiden Schriften sind Scharniere gesondert dargestellt. Dies aber nur, um die Funktion der Scharnier klarzumachen. Bei Anwendung auf im Spritzguß hergestellte Behälter oder Flaschen wurden stets die Scharniere zusammen mit dem Behälter in einem Arbeitsgang gespritzt. Dies gestattet nur das Spritzen relativ einfach gebauter Behälter oder würde sehr komplizierte Spritzformen erfordern.

Durch die vorliegende Erfindung soll ein Schnappscharnier der genannten Art geschaffen werden, das die Herstellung auch kompliziert geformter Behälter im Spritzgußverfahren ohne komplizierte Formen gestattet. Das Spritzen von Behälter und Scharnier soll vereinfacht und verbilligt werden. Es soll die Möglichkeit geschaffen werden, mit Schnappscharnieren ausgestattete Behälter im Srritzgußverfahren herzustellen, die wegen der Kompliziertheit ihrer Formen bisher nicht oder nur mit hohem technischen Aufwand herstellbar waren.

0167661
zel-65-/lcp

Diese Aufgabe wird nach Anspruch 1 gelöst.

Hiernach wird das Schnappscharnier als gesonderter Bauteil gefertigt, was mit einfachen Formen möglich ist, und der eine der Scharnierteile ist mit Steckteilen zum Befestigen am Behälter oder an einem Verschlußstück des Behälters versehen.

Beim Bau der Spritzformen für den Behälter selbst oder sein Verschlußstück braucht infolgedessen nicht mehr auf die Ausbildung des Schnappscharniers Rücksicht genommen zu werden. Vielmehr werden verschiedene Bauteile getrennt hergestellt, einerseits das Schnappscharnier, andererseits der Behälter (meist eine Flasche) und gegebenenfalls ein am Behälter anzubringendes Verschlußstück.

## Weiterbildungen der Erfindung

Eine besonders günstige Bauform für ein Schnappscharnier ergibt sich aus Anspruch 2. Wird nämlich das bogenförmige oder winkelförmige federnde Verbindungsstück am Schnappscharnier so angeordnet, daß es in Schließstellung gegen den Behälter weist, so tritt es bei geschlossenem Behälter nach außen nicht·störend in Erscheinung. Die Außenfläche des Schnappscharniers ist in Schließstellung z. B. eine Ebene (oder eine dem Behälter angepaßte gewölbte Fläche), die das Hauptgelenk enthält, so daß sich ein befriedigender ästhetischer Eindruck ergibt. Es stehen keine Scharnierteile nach außen störend vor.

Nach Anspruch 3 lassen sich die Gelenke dann besonders günstig anordnen, wenn die beiden Scharnierteile plattenförmig ausgebildet sind und die Platten eine ausreichende Dicke haben.

Bei dünneren Platten können nach Anspruch 4 die Nebengelenke an vorspringenden Leisten der Scharnierteile ansetzen.

Nach Anspruch 5 kann der eine Scharnierteil einen oder mehrere

0167661
zel-65-Aep

Steckzapfen mit Außenwulst haben, die zum Einstecken in entsprechende Öffnungen am Behälter oder seinem Verschlußstück dienen.

Nach Anspruch 6 kann der eine Scharnierteil dagegen mindestens eine Steckhülse haben, die einen Innenwulst aufweist und zum Aufstecken auf einen entsprechenden Zapfen am Behälter oder seinem Verschlußstück dient.

Nach Anspruch 7 kann eine solche Steckhülse so ausgebildet sein, daß sie bei aufgestecktem Schnappscharnier die Mündung des Behälters umgibt, während der andere Scharnierteil so angeordnet ist, daß er beim Verschließen der Mündung in diesen Rohrstutzen hineinschnappt.

Ausführungsbeispiele mit weiteren Merkmalen der Erfindung werden im folgenden anhand der Zeichnungen beschrieben.

Figur 1   ist eine Ansicht einer ersten Ausführungsform eines Schnappscharniers nach der Erfindung von unten.

Figur 2   ist eine Seitenansicht desselben Schnappscharniers.

Figur 3   ist ein mittiger Längsschnitt durch dieses Schnappscharnier.

Figur 4   ist ein mittiger Längsschnitt durch den oberen Teil eines durch das Schnappscharnier zu verschließenden Behälters.

Figur 5   zeigt das Schnappscharnier in Seitenansicht nach Figur 2, jedoch geöffnet.

Figur 6   zeigt ein Schnappscharnier nach einer zweiten Ausführungsform der Erfindung in mittigem Längsschnitt.

Figur 7   zeigt dasselbe Schnappscharnier in Draufsicht von außen (oben).

Figur 8   zeigt in mittigem Längsschnitt ein Verschlußstück zur Aufnahme eines Schnappscharniers nach Figur 6 und 7 und zum Anbringen an einem Behälter.

Figur 9   ist eine Draufsicht auf die Hälfte dieses Verschlußstückes von oben.

Figur 10 zeigt eine dritte Ausführungsform eines Schnappscharniers nach der Erfindung in Seitenansicht.

Figur 11 ist eine Draufsicht auf dieses Schnappscharnier.

Figur 12 ist ein mittiger lotrechter Querschnitt durch dieses
    Schnappscharnier.

Figur 13 ist eine Einzelheit dieses Schnappscharniers und zeigt
    die Gelenke.

Figur 14 (Blatt 2) ist eine Seitenansicht entsprechend Figur 10,
    jedoch in Offenstellung des Schnappscharniers.

In allen Ausführungsbeispielen beziehen sich Begriffe wie "oben",
"unten" und dergleichen immer auf die Lage eines Schnappscharniers an einem aufrechtstehenden Behälter, bei dem die Mündung
aufwärts gerichtet ist.

Alle dargestellten Schnappscharniere werden einteilig, z. B. aus
Polypropylen gespritzt.

## Erste Ausführungsform

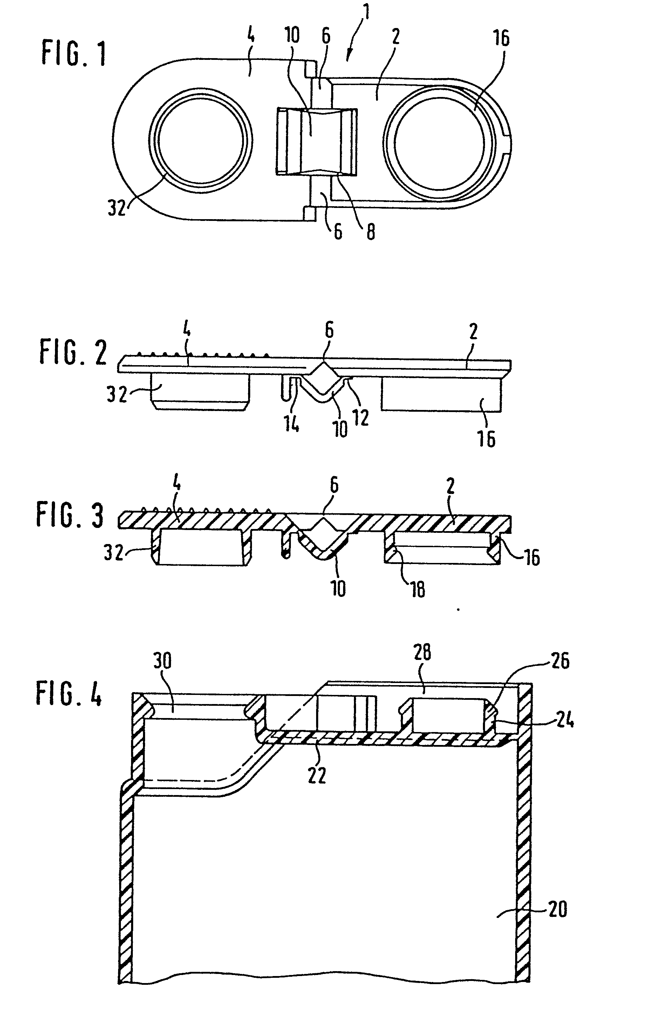

Das in Figur 1 bis 3 und 5 dagestellte Schnappscharnier 1 hat
zwei plattenartige Scharnierteile 2 und 4, die durch ein als
Filmscharnier ausgebildetes Hauptgelenk 6 miteinander verbunden
sind. Das Hauptgelenk ist zweiteilig ausgebildet. Zwischen seinen
beiden Teilen befindet sich eine Öffnung 8, in der ein federndes
Verbindungsstück 10 untergebracht ist. Das Verbindungsstück 10
steht durch zwei Nebengelenke 12 und 14 mit den Scharnierteilen 2
und 4 in Verbindung. An dem Scharnierteil 2 ist eine Steckhülse
16 vorgesehen, die mit einem Innenwulst 18 versehen ist.

An einem Behälter 20 (Figur 4), von dem nur das obere Ende gezeigt
ist, befindet sich (auf einer oberen Abschlußplatte 22) eine
Steckhülse 24, die hier als Steckzapfen fungiert und einen Außenwulst 26 besitzt. Der Scharnierteil 2 läßt sich mit Hilfe seiner
Steckhülse 16 auf der Steckhülse 24 des Behälters verankern. Eine
die Abschlußplatte 22 umlaufende Wand 28 des Behälters sorgt für gleichbleibende Orientierung des Schnappscharniers 1.

Der Behälter hat links eine Entnahmeöffnung 30. An dem Scharnierteil 4 ist ein dazu passendes Verschließelement 32 in Form eines
Rohrstutzens vorgesehen. Am Scharnierteil 4 befindet sich außerdem ein plattenartiger Teil 34 von der Breite des federnden Verbindungsstücks 10.

In den Figuren der folgenden Ausführungsformen sind Bauteile
gleicher oder ähnlicher Funktion wie bei der ersten Ausführungsform mit denselben Bezugzeichen wie dort versehen.

## Zweite Ausführungsform

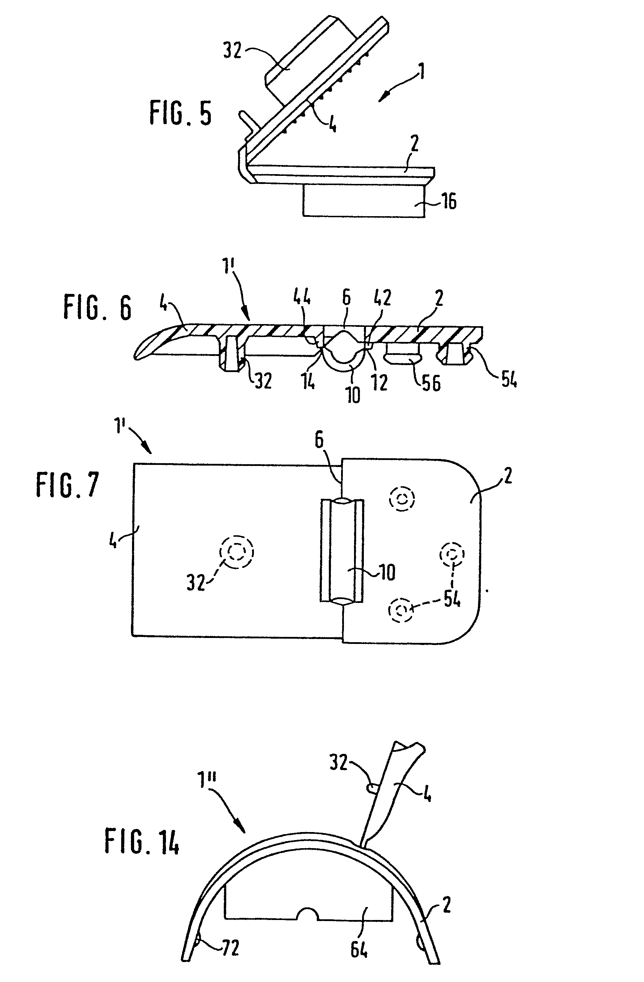

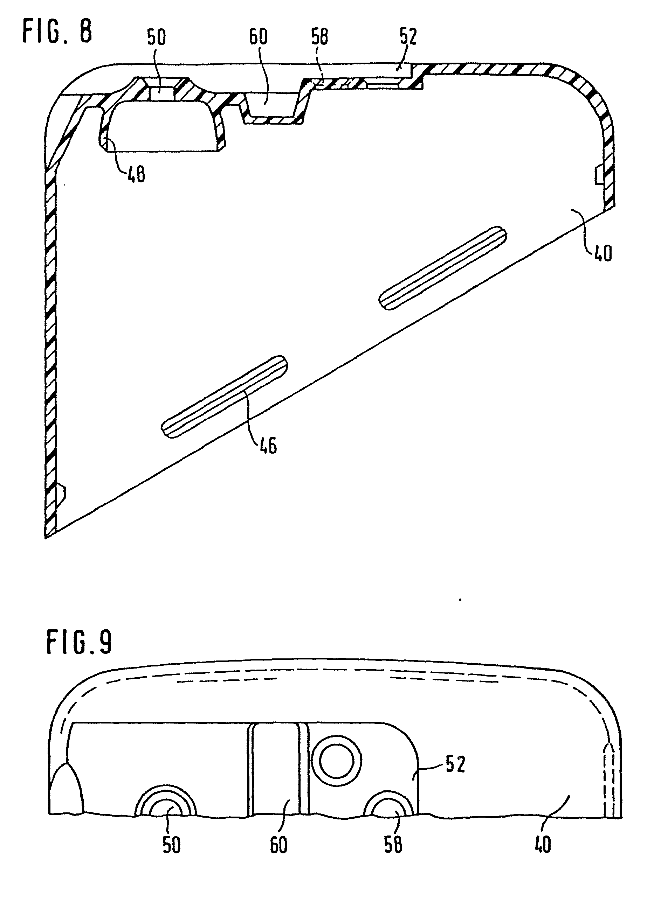

Die Figuren 6 und 7 zeigen ein Schnappscharnier 1`, das nicht
unmittelbar an einem Behälter sondern an einem Verschlußstück
nach Figur 8 und 9 anzubringen ist, das seinerseits auf einem
Behälter zu befestigen ist. Während das federnde Verbindungsstück
10 bei der ersten Ausführungsform gewinkelt ist, ist es hier
bogenförmig ausgebildet. Es hat aber die gleiche Funktion. Die
beiden Nebengelenke 12 und 14 sitzen nach Figur 6 nicht unmittelbar an den Scharnierteilen 2 und 4 sondern an Leisten 42 und 44,
die von den Scharnierteilen 2 und 4 nach unten vorspringen.

Das in Figur 8 und 9 dargestellte Verschlußstück 40 ist als Kappe
mit schräger unterer Berandung ausgebildet. Die Kappe hat nahe
ihrem unteren Rand Schnappwülste 46, die in entsprechende Vertiefungen eines nicht dargestellten Behälters nach dem Aufstecken
einrasten. Das Verschlußstück hat oben links innen einen Napf 48,
der die Behältermündung umgeben soll, und zentrisch dazu eine
Austrittsöffnung 50. Am Schnappscharnier 1` ist ein dazu passendes Verschließelement 32 in Form eines hohlen Zapfens vorgesehen.

Das Verschlußstück hat oben eine flache Ausnehmung 52 zur Aufnahme des Schnappscharniers. An dem Scharnierteil 2 sind drei Steckzapfen 54 vorgesehen, die unten je einen äußeren Ringwulst 56
haben. Die Steckzapfen lassen sich in durchgehende Öffnungen 58
des Verschlußstückes 40 eindrücken, sind durch ihre Ringwülste 56
gegen Herausrutschen gesichert und haltern den Scharnierteil 2 in
der flachen Ausnehmung 52 des Versschlußteils.

Zur Unterbringung des federnden Verbindungsstückes 10 ist in der
Deckplatte des Verschlußstückes eine Vertiefung in Form einer
quer verlaufenden Rinne 60 vorgesehen.

Die Offenstellung des Schnappscharniers sieht hier ähnlich aus
wie die nach Figur 5.


## Dritte Ausführungsform

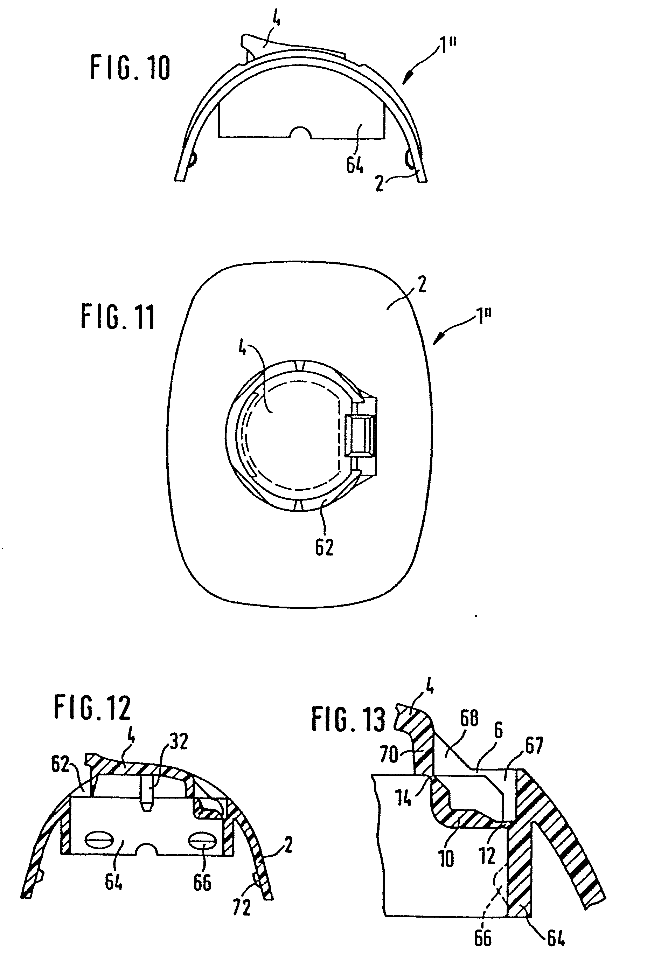

Das in den Figuren 10 bis 14 (Figur 14 auf Blatt 2) dargestellte
Schnappscharnier 1" hat wiederum einen Scharnierteil 4, der mit
einem Verschließelement 32, hier in Form eines Zapfens, für eine
Ausspritzöffnung einer Flasche versehen ist. Im Gegensatz zu den
beiden vorangehenden Ausführungsformen ist der Scharnierteil 4
jedoch innerhalb einer Öffnung 62 des Scharnierteils 2 untergebracht. Der Scharnierteil 2 dient als ganzer als Steckteil und
hat Haltenocken 72. Außerdem ist eine Steckhülse 64 eingeformt,
die innenliegende Nocken 66 hat und zum Aufstecken auf die Mündung einer Flasche dient. Das Hauptgelenk 6 befindet sich zwischen  Vorsprüngen 67 (die von der Steckhülse 64 geringfügig
einwärts ragen) und Vorsprüngen 68 des Scharnierteils 4. Das
federnde Verbindungsstück 10 sitzt mit seinem Nebengelenk 12 an
der Innenwandung der Steckhülse 64 und mit seinem Nebengelenk 14
an einer geraden kurzen Seitenwand 70 des Scharnierteils 4.


- - - - - - -

BEZUGSZEICHEN

| | | |
|---|---|---|
| 1, 1´, 1" | | Schnappscharnier |
| 2, 4 | | Scharnierteil |
| 6 | | Hauptgelenk |
| 8 | | Öffnung |
| 10 | | federndes Verbindungstück |
| 12, 14 | | Nebengelenk |
| 16 | | Steckhülse |
| 18 | | Innenwulst |
| 20 | | Behälter |
| 22 | | Abschlußplatte |
| 24 | | Steckhülse |
| 26 | | Außenwulst |
| 28 | | Wand |
| 30 | | Entnahmeöffnung |
| 32 | | Verschließelement |
| 40 | | Verschlußstück |
| 42, 44 | | Leiste |
| 46 | | Schnappwulst |
| 48 | | Napf |
| 50 | | Austrittsöffnung |
| 52 | | Ausnehmung |
| 54 | | Steckzapfen |
| 56 | | äußerer Ringwulst |
| 58 | | Öffnung |
| 60 | | Rinne |
| 62 | | Öffnung |
| 64 | | Steckhülse |
| 66 | | Nocken |
| 67, 68 | | Vorsprung |
| 70 | | Seitenwand |
| 72 | | Haltenocken |

- - - - - -

PATENTANSPRÜCHE

1. Einteiliges Schnappscharnier aus Kunststoff mit folgenden Merkmalen:

a) Zwei Scharnierteile sind durch ein Hauptgelenk miteinander verbunden;

b) mindestens ein federndes Verbindungsstück ist an seinen beiden Enden durch Nebengelenke mit beiden Scharnierteilen verbunden;

c) die Nebengelenke haben quer zu den Gelenkachsen je einen Abstand vom Hauptgelenk;

d) die Gelenke sind als Filmscharniere ausgebildet;

e) derart, daß die Scharnierteile die Tendenz haben, aus einer Stellung labilen Gleichgewichts, die sich innerhalb ihres Schwenkbereichs befindet, in die eine oder andere Endstellung zu schnappen,

gekennzeichnet durch folgende Merkmale:

f) Das Schnappscharnier (1, 1´, 1") ist ein gesonderter Bauteil;

g) der eine Scharnierteil (4) dieses Bauteils trägt ein Verschließelement (32) für eine Entnahmeöffnung eines Behälters, insbesondere einer Flasche oder eines Verschlußstückes des Behälters;

h) der andere Scharnierteil (2) trägt zum Befestigen am Behälter oder an seinem Verschlußstück mindestens einen
Steckteil mit Rastelement(en).

2. Schnappscharnier nach Anspruch 1, **gekennzeichnet** durch folgende Merkmale:

a) das federnde Verbindungsstück (10) ist bogenförmig oder
winkelförmig ausgebildet und weist in Schließstellung des
am Behälter oder Verschlußstück angebrachten Schnappscharniers gegen den Behälter;

b) das Hauptgelenk (6) ist an der vom Behälter abgekehrten
Seite des Schnappscharniers angeordnet.

3. Schnappscharnier nach Anspruch 2, **gekennzeichnet** durch folgende Merkmale:

a) die Scharnierteile (2, 4) sind im wesentlichen plattenförmig;

b) das Hauptgelenk (6) einerseits und die Nebengelenke (12,
14) andererseits befinden sich an gegenüberliegenden Flächen der Scharnierteile.

(Figur 2 und 3)

4. Schnappscharnier nach Anspruch 3, dadurch gekennzeichnet, daß
die Nebengelenke (12, 14) an Leisten (42, 44) sitzen, die von
den Scharnierteilen gegen den Behälter vorspringen.

5. Schnappscharnier nach Anspruch 1, dadurch gekennzeichnet, daß
der Steckteil als Steckzapfen (54) mit äußerem Ringwulst (56)
ausgebildet ist. (Figur 6)

6. Schnappscharnier nach Anspruch 1, dadurch gekennzeichnet, daß der Steckteil als Steckhülse (16; 64) mit innerem Ringwulst (18) oder innen liegenden Nocken (66) ausgebildet ist. (Figur 3; 12)

7. Schnappscharnier nach Anspruch 6, gekennzeichnet durch folgende Merkmale:

   a) die Steckhülse (64) dient zugleich zur Aufnahme der Behältermündung;

   b) der das Verschließelement tragende Scharnierteil (4) ist mit seinem Hauptgelenk (6) und einem Nebengelenk (12) mit der Steckhülse (64) verbunden und liegt in Schließstellung zumindest teilweise im Innern der Steckhülse.

(Figur 10 bis 13)

- - - - -

**FIG. 1**

**FIG. 2**

**FIG. 3**

**FIG. 4**

FIG. 5

FIG. 6

FIG. 7

FIG. 14

**FIG. 8**

50    60    58    52

48

40

46

**FIG.9**

52

50    60    58    40

0167661

FIG. 10

FIG. 11

FIG. 12

FIG. 13

Europäisches
Patentamt

**EUROPÄISCHER RECHERCHENBERICHT**

**0167661**
Nummer der Anmeldung

EP 84 11 1399

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 4) |
|---|---|---|---|
| Y,D | DE-A-1 808 875 (AMERICAN OPTICAL CORP.)<br>* Figuren 7-12; Seite 2, Abschnitt 2; Seite 8, Zeilen 5-19 * | 1,2,5 | B 65 D 47/08<br>E 05 D 1/02 |
| Y | US-A-3 493 150 (M.B. LUCAS & D.L. CRANE)<br>* Figuren 1,3; Spalte 3, Zeilen 34-61; Spalte 4, Zeilen 39-45 * | 1,2,5 | |
| A | BE-A- 689 387 (WESTHEM CORPORATION)<br>* Figur 4; Seite 3, letzter Abschnitt; Seite 9, Abschnitt 3 * | 3 | |
| A | FR-A-2 288 683 (STE L'OREAL)<br>* Figuren 1-5; Seite 5, Zeile 40 - Seite 6, Zeile 5; Seite 7, Zeile 10 - Seite 8, Zeile 1 * | 6,7 | RECHERCHIERTE SACHGEBIETE (Int. Cl.4)<br><br>B 65 D<br>E 05 D<br>E 05 F |

Der vorliegende Recherchenbericht wurde für alle Patentansprüche erstellt.

| Recherchenort<br>DEN HAAG | Abschlußdatum der Recherche<br>26-09-1985 | Prüfer<br>SCHEIBLING C.D.A. |
|---|---|---|